# EUROPEAN PATENT SPECIFICATION

(11) **EP 1 722 678 B1**
(45) Date of publication and mention of the grant of the patent: **30.01.2008**
(21) Application number: 05715478.3
(22) Date of filing: 23.02.2005
(51) Int. Cl.: A61B 5/024, A61B 5/0444

(54) **ASSESSMENT OF FETAL REACTIVITY BY FETAL HEART RATE ANALYSIS**
BEURTEILUNG DER FÖTALEN REAKTIVITÄT DURCH FÖTALE HERZFREQUENZANALYSE
EVALUATION DE LA REACTIVITE FOETALE PAR L'ANALYSE DE LA FREQUENCE CARDIAQUE FOETALE

(30) Priority: 24.02.2004 EP 04004114
(43) Date of publication of application: 22.11.2006
(73) Proprietor: Neoventa medical AB, 431 35 Mölndal (SE)
(72) Inventor: OUTRAM, Nicolas, Tavistock PL19 8DB (GB); ROSEN, Karl Gustaf, S-442 35 Kungälv (SE)
(74) Representative: Edlund, Fabian
(86) International application number: PCT/EP2005/001887
(87) International publication number: WO 2005/079666

(56) References cited:
- KR-A- 20030 069 586
- US-A- 4 510 944
- US-A- 5 042 499
- SIBONY O ET AL: "QUANTIFICATION OF THE FETAL HEART RATE VARIABILITY BY SPECTRAL ANALYSIS OF FETAL WELL-BEING AND FETAL DISTRESS" EUROPEAN JOURNAL OF OBSTETRICS & GYNECOLOGY AND REPRODUCTIVE BIOLOGY, EXCERPTA MEDICA, AMSTERDAM,, NL, vol. 54, no. 2, April 1994 (1994-04), pages 103-108, XP009041776 ISSN: 0301-2115

## Description

### TECHNICAL FIELD OF THE INVENTION

The present invention relates to the field of fetal heart rate (FHR) monitoring. In particular, it relates to monitoring during periods of so-called non-stationary fetal heart rate patterns, such as those that occur during labour.

### BACKGROUND

The fetal heart rate is an important indicator of fetal health during pregnancy and labour. Some variability in the heart rate is desirable, as this shows that the fetus is responding to stimuli. However, certain changes can also indicate that the fetus is experiencing distress and may be suffering from the early stages of hypoxia (lack of oxygen).

It is common practice for the FHR to be measured during labour. At present, the FHR is normally presented as a plot against time on a monitor. It is then studied visually by medical personnel who check for signs of fetal distress. Thus, the system relies upon the skill and experience of the user.

During gestation and birth the fetus alternates between sleep states and active states and these are indicated by a change in FHR. These changes occur suddenly and each state lasts for a period of up to 60 minutes, depending on the development of the fetus. An experienced obstetrician is able to observe these alterations and determine whether these are normal for the stage and development of the fetus.

During labour, the fetus is subject to additional influences, such as uterine contractions, that may significantly alter its FHR by means of intermittent umbilical cord compression, mechanical pressure etc. In addition, other factors which affect the heart rate, such as oxygenation, drugs, fetal breathing activity, glucose levels etc, can vary significantly during labour in a manner which is not witnessed previously during pregnancy. This results in comparatively long term changes in the FHR, as opposed to the abrupt shifts caused by a change in state, and creates what is called a non-stationary heart rate.

These changes mean that a non-stationary heart rate is much harder to decipher, even for an experienced obstetrician. Often, early warning signs provided by anomalies in the FHR beat-to-beat variation are misinterpreted or missed altogether. This can result in severe intrapartum hypoxia. Alternatively, upon viewing an abnormal FHR reading, these (normal) changes may cause obstetricians to err on the side of caution which results in unnecessary surgical intervention.

In order to assist in the correct identification of fetal distress, the present applicants have developed the STAN® fetal monitoring system. Its methodology is based upon the combination of FHR monitoring and ST waveform analysis of the fetal ECG. The system automatically detects abnormalities in the ST waveform (called ST events) that are indicative of hypoxia. The ST events are in effect used as an alarm to allow the operator to focus on the FHR patterns.

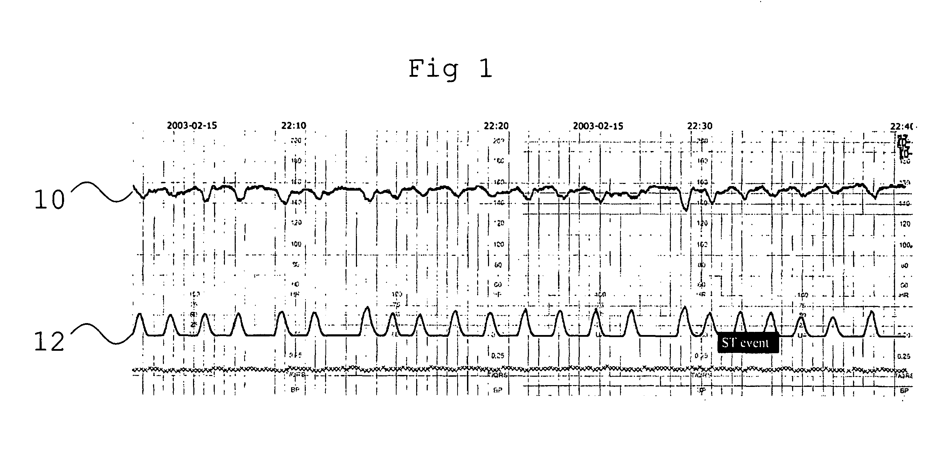

Potential problems with the fetus are indicated by extremes in FHR variation. FIG 1 shows a 35 minute STAN® recording in a 1^{st} stage of labour in which the fetus is exposed to slowly developing hypoxia. The recording shows the FHR 10 together with the uterine activity 12. Here, the fetus is showing signs of reduced beat-to-beat variation and no reactivity (i.e. no response to stimuli). Although such a pattern can normally be identified visually, there are situations where this may be missed if no other warnings are given by additional medical equipment. Further, when the FHR variability gradually decreases over time, these slight alterations may be overlooked.

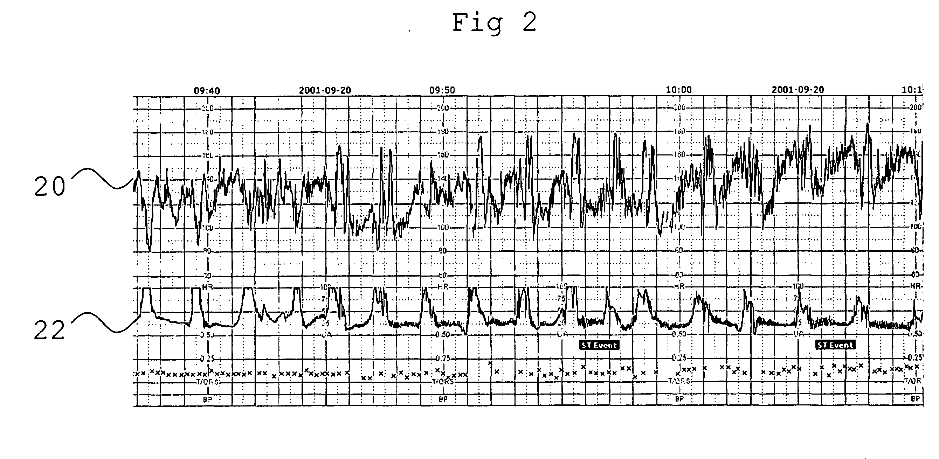

FIG 2 shows an incidence of increased FHR variation. Again, the FHR 20 is shown together with uterine activity 22. This recording was taken during the last stages of labour when is it normal for large variations in FHR to occur. However, in this instance the increased variation was a response to developing hypoxia. In situations such as these, it is not uncommon for the FHR to be misread as normal, or at least ambiguous. This can lead to delays which may severely affect the health of the baby.

There therefore exists a need for a method of more clearly analysing FHR variations, in particular during labour, such that fetal distress (hypoxia) can be correctly diagnosed earlier.

Previously, attempts have been made to quantify FHR variation by standard statistics (analysis of variance, standard deviation etc) and more sophisticated power spectrum analysis. Several studies have been conducted on spectral analysis of the FHR as a method to quantify changes over time in FHR variation. One such study is discussed in "Quantification of the fetal heart rate variability by spectral analysis of fetal well being and fetal distress", Akselrod S et al, Eur J Obstet Gynecol Reprod Biol 1994; 54; 103-8. However, spectral analysis requires a continuous and stationary sequence of FHR data and does therefore not lend itself to continuous assessment of FHR during labour, when the FHR tends to be non-stationary. Consequently, these methods have been of little use during labour.

Computerised analysis of FHR variation has also been applied to FHR tracings obtained prior to the onset of labour. Sonicaid 8000 is a system currently being marketed to assist in the quantification of FHR variation before labour starts ("Antenatal cardiotocogram quality and interpretation using computers" Dawes GS et al, Br J Obstet Gynaecol. 1992 Oct; 99(10): 791-7). This system is based on applying standard techniques of FHR beat-to-beat variance measurements (mean values, standard error of the mean etc) and again does not lend itself to continuous FHR variation assessments in a non-stationary FHR signal. Thus, computerised assessment of FHR beat-to-beat variations has not proved helpful during labour.

Consequently, FHR variation during labour is still conducted visually by a skilled, experienced medical practitioner, which can occasionally lead to signs of fetal distress being overlooked or wrongly diagnosed, as discussed above.

### SUMMARY OF THE INVENTION

It is therefore an object of the present invention to mitigate limitations related to the background art described above.

A further object of the present invention is to provide an improved apparatus, method and computer program for more clear analysis of FHR variations, in particular during labour, such that fetal distress (hypoxia) can be correctly diagnosed earlier.

Another object of the present invention is to provide an improved computerised assessment of FHR variations during labour.

According to a first aspect of the present invention, there is provided an apparatus for fetal monitoring comprising:
means for determining a fetal heart rate,
means for identifying a primary fetal heart rate component which is required to shift a volume of blood from the heart to the cardiovascular system, and
means for subtracting the primary component from the determined fetal heart rate to determine a residual component; and
means for using said residual component for analysis of the fetal heart rate beat-to-beat variation.

The invention is based upon recognition by the inventors that the FHR can be separated into two elements. Firstly, there is the FHR component which is required to allow the heart to serve as a pump and shift a volume of blood to the cardiovascular system. This is defined as the primary FHR component. As well as this basic requirement, as previously discussed, the FHR is under the influence of numerous other sources. These secondary FHR components, most of which are under the influence of higher central nervous system structures, are termed the FHR residual component and comprise the remainder of the FHR.

It is this FHR residual component which provides a measure of external influences and, most importantly, the ability of the central nervous system to react and respond to changes in the internal milieu not primarily associated with the need to shift blood volume. By identifying this, the invention allows indications of fetal distress to be more readily identified.

The present invention also extends to a corresponding method and therefore viewed from another aspect the invention provides an method for fetal monitoring comprising:
determining a fetal heart rate,
identifying a primary fetal heart rate component which is required to shift a volume of blood from the heart to the cardiovascular system, and
subtracting the primary component from the determined fetal heart rate to determine a residual component; and
using said residual component for analysis of the fetal heart rate beat-to-beat variation.

The FHR residual component, r, can be isolated by identifying the primary FHR component, p, and subsequently subtracting this from the recorded FHR, y, leaving only the FHR residual. This can be expressed mathematically by the equation r = y - p.

The FHR residual component can then be analysed to provide information on the reactivity of the fetus and thus its health. This may be done visually, for example by studying a plot on a monitor, but preferably statistical tests are applied. For example, the frequency distribution of residual measurements, median, variance, 95^{th} percentile, etc may be determined.

In order to obtain useful results, it is clearly important to correctly identify the FHR primary component. Several techniques can be used to achieve this, such as integration of the recorded data over a predetermined time interval. However, it has been found that it is preferable to identify the primary FHR component through a polynomial curve fit approximation of the recorded data.

This is particularly useful during labour when the non-stationary nature of the FHR makes other methods, such as integration, unreliable. In these circumstances the approximation should preferably be instantaneous and therefore a polynomial curve fit provides the best results. Furthermore, a polynomial curve fit would allow for the primary FHR component to be calculated despite intermittent loss of FHR data, a constant feature during the final stage of delivery.

The means for identifying the primary fetal heart rate component, comprised in the apparatus according to the present invention may be adapted to perform the following steps:
i) linear interpolation of recorded fetal heart rate data;
ii) resampling at a resampling frequency, thereby forming a resampled series of fetal heart rate data, and;
iii) polynomial curve fit approximation of said resampled series.

After linear interpolation of consecutive heart beats, the event series can be resampled at a resampling frequency. The resampling frequency may be higher than the actual heart rate, for example a resampling frequency of 4 Hz may be used. An effect of performing a polynomial curve fit approximation of a resampled series is that the problem of under-fitting at low heart rates may be reduced.

The polynomial curve fit approximation may utilise polynomials of at least the 5^{th} order. Especially, polynomials of the 5^{th} order or polynomials of the 12^{th} order may preferably be used.

Through experimentation with previously recorded FHR variation data, it has been found that it is preferable to use a least squares polynomial approximation.

A least squares approximation may be formulated by minimising the function |**P**x-y|², where y is a vector representative of n heart rate samples, **P** is an NxM matrix where the columns are discrete independent polynomial functions and x is a vector of real valued coefficients. The well-known least squares solution for the coefficient vector is x=(**P**^{T}**P**)⁻¹**P**^{T}y. The approximation of the primary FHR component, p, will therefore be the projection of y onto the subspace spanned by **P**, given by p=**P**x=**P**(**P**^{T}**P**)⁻¹**P**^{T}y. The success of this projection, for any given application, depends on how much the desired solution space is spanned by the subspace defined by **P**.

However, making the best choice of **P** can be difficult for some functions, in particular in the case of biomedical signals such as the heart rate as there is no *a priori* information on the nature of the function. It is known that the heart rate has regions of high and low frequencies within any interval of interest. Such a situation can lead to a "ringing" phenomenon when using polynomial approximations, thus causing inaccurate results.

One preferred solution is to divide the data into small adjacent regions and perform independent polynomial approximations in each region. This improves the accuracy of the result as there are then a guaranteed maximum number of degrees of freedom (turning points) in each region. Furthermore, as each approximation is independent of the others, they will not induce "ringing".

As each approximation is independent, the polynomial curves may not align at the region boundaries. To overcome this and achieve a continuous function, it is preferable to apply constraints to the formulation of each approximation. These constraints are preferably that neighbouring polynomial functions must align and have equal first derivatives (i.e. gradients) at the region border where they join. The constraints may be applied using the well-known method of Lagrange multipliers. It can be shown that by formulating these constraints with known polynomials, e.g. Legendre or Chebyshev, new discrete and independent polynomials can be derived that more closely span the desired solution space.

Viewed from another aspect therefore, the present invention provides a method for determining the primary FHR component in a recording of fetal heart rate beat to beat variation, comprising the steps of dividing the recording into regions of a predetermined size and performing individual polynomial approximations in each region, wherein each polynomial approximation is constrained such that neighbouring polynomial functions align and have equal first derivatives at the region border where they join.

By visual inspection and empirical testing, it has been found that an excellent primary FHR variation approximation can be achieved through applying consecutive constrained 5^{th} or 12^{th} order Legendre polynomial approximations, each spanning at least 20 FHR samples.

Following the identification of the primary FHR component, the FHR residual component can be determined by subtracting the primary component from the determined fetal heart rate. In order to further improve the quality of the residual data, it may be normalised with respect to a baseline fetal heart rate.

The means for using the residual component for analysis of the fetal heart rate beat-to-beat variation, comprised in the apparatus according to the present invention, may be adapted to apply statistical tests for analysing the residual component in order to determine the response of the fetus. The statistical tests may comprise calculating a median and a variance of the 95^{th} percentile over a predetermined period of time. The predetermined period of time may be longer than 10 minutes.

The means for using the residual component for analysis of the fetal heart rate beat-to-beat variation, comprised in the apparatus according to the invention, may be adapted to class the fetal heart rate as abnormal and non-reactive if the median of the 95^{th} percentile is consistently below 3ms.

It has been found that consistent readings of a running 20 minutes median value of <3ms indicate a low FHR variation giving rise to concern for the wellbeing of the fetus.

The above-mentioned means for using the residual component for analysis of the fetal heart rate beat-to-beat variation may be adapted to indicate a significant reduction of fetal reactivity given a recording of the median of the 95^{th} percentile below 2.3 ms and the variance of the 95^{th} percentile below 0.1 over an extended period of time.

The extended period of time may be longer than 10 minutes. Tests have shown that epoques exceeding 60 minutes during which the median of the 95^{th} percentile is below 2.3 ms and the variance is below 0.1 would identify an adverse fetal state with a sensitivity of 100%.

The above-mentioned means for using the residual component for analysis of the fetal heart rate beat-to-beat variation may be adapted to indicate a significant reduction of fetal reactivity given a recording of a decreasing trend of the median of the 95^{th} percentile over an extended period of time.

In this case, the extended period of time may be longer than 30 minutes. Tests have shown that a progressive fall in the median of the 95^{th} percentile, recorded over 20 minute intervals, over a two hour recording sequency would identify an abnormal fetal response to the stress of labour.

The above-mentioned means for using the residual component for analysis of the fetal heart rate beat-to-beat variation may be adapted to indicate an abnormally low fetal heart rate variation if the median of the 95^{th} percentile is consistently above 3ms.

Furthermore, it has been found that a frequency distribution within the 3-4ms domain with periods of >7% indicates a healthy level of reactivity. This function is based on the observation that a fetus, although in a sleep state with low beat-to-beat variation, will express intermittent bursts of activity as detected by an increase in instantaneous beat-to-beat variation. The conventional method of assessing reactivity features is to visually identify episodic accelerations. This is believed to be inventive in itself and so, viewed from another aspect, the invention provides a method of indicating the possibility of fetal distress comprising the steps of: obtaining FHR residual component data, providing a reading of the 95^{th} percentile and providing a reading of the 3-4ms frequency distribution. Table I gives the characteristics of the residual measurements required to assess a normal, healthy fetus with a reactive FHR trace, a fetus unable to respond to the stress of labour (Preterminal) as well as the fetus forced to respond with an extraordinary regulatory effort (Alarm reaction).

In Table I, FD₃₋₄ₘₛ means the frequency distribution of FHR residual measurements recorded within the 3-4ms domain, and 95^{th} 20' median means the 95^{th} percentile of residual measurements recorded as a running 20 minutes median value.

**Table I: Criteria to indicate preterminal FHR pattern and alarm reaction from FHR residual measurements.**

| | | | |
|---|---|---|---|
| Residual measurements | Observations made during the first 30 minutes of a recording | | |
| | Normal at onset | Ambiguous at onset | Preterminal status at onset |
| | FD₃₋₄ₘₛ consistently >3% or 1-3% range but with episodes of >7.0% | All other observations. Accelerations will indicate normality. | FD₃₋₄ₘₛ consistently <1% in a 10' block of data or consistently <2% during the 30 minutes |
| | Observations made after the first 30 minutes of a recording to indicate a preterminal fetal heart rate pattern | | |
| Residual measurements | 95^{th} _{20' median} consistently below 3 ms for 90 min | 95^{th} _{20' median} consistently below 3 ms for 60 min | 95^{th} _{20' median} consistently below 3 ms for 60 min |
| | 95^{th} _{20' median} consistently below 3 ms for 40' and FD₃₋₄ₘₛ consistently <1% in a 20' block of data | 95^{th} _{20' median} consistently below 3 ms for 40' and FD₃₋₄ₘₛ consistently <1% in a 20' block of data | 95^{th} _{20' median} consistently below 3 ms for 20' and FD₃₋₄ₘₛ consistently <1% in a 20' block of data |
| | | Note: FD_{3-4 raw} >7.0% for >2' will automatically transfer the case to a Normal status. | |
| | Observations to indicate an Alarm reaction | | |
| Residual measurements | 95^{th} _{20' median} > 25 ms for > 6 minutes | | |

Preferably the FHR residual data is provided as discussed above. The invention also extends to a monitoring apparatus arranged to perform this method.

According to another aspect of the present invention it extends to a computer program for executing the steps of:
determining a fetal heart rate,
identifying a primary fetal heart rate component which is required to shift a volume of blood from the heart to the cardiovascular system, and
subtracting the primary component from the determined fetal heart rate to determine a residual component; and
using said residual component for analysis of the fetal heart rate beat-to-beat variation.

### BRIEF DESCRIPTION OF THE DRAWINGS

An embodiment of the invention will now be described, by way of example only, with reference to the accompanying figures, in which:
Figure 1 shows a 35 minute STAN^{®} recording in 1^{st} stage of labour in which the fetus is exposed to slowly developing hypoxia;
Figure 2 shows a STAN^{®} recording during the last stages of delivery showing the FHR overacting with an "Alarm reaction" to developing hypoxia;
Figure 3 shows a plot of FHR variation with the identified primary fetal heart rate component superimposed;
Figure 4A shows a plot of the FHR with the identified primary FHR component superimposed;
Figure 4B shows the resulting FHR residual component of FIG 4A;
Figures 5 A and B respectively show the FHR and a plot of the 95^{th} percentile of the FHR residual component in a situation where the fetus is displaying a lack of reactivity;
Figure 6 shows a plot of the frequency distribution of 3-4ms FHR residuals in a fetus showing a lack of reactivity;
Figures 7A and B respectively show the FHR and a plot of the 3-4ms frequency distribution in an instance where the fetus is gradually losing reactivity;
Figures 8A and B respectively show the FHR and a plot of the 3-4ms frequency distribution in an instance where the FHR data alone is ambiguous;
Figure 9 shows a plot of the 95^{th} percentile of the FHR residual component in respect of the fetus recorded in figure 2.
Figure 10 provides a flow chart indicating the different steps in the residual analysis process according to one embodiment of the invention.
Figure 11 is a schematic illustration of the apparatus according to one embodiment of the present invention.

### DETAILED DESCRIPTION

During labour the FHR is monitored for signs of fetal distress (lack of oxygen). Figure 1 shows a 35 minute STAN^{®} recording of a 1st stage of labour. The case illustrates a situation of lack of FHR variability and reactivity (preterminal FHR pattern) that at time of the recording was not recognised in spite of the ST event. The figure also depicts low measurements of "residual" markers of FHR reactivity and variability (Frequency distribution within the 3-4ms range of residual measurements (FD₃₋₄ₘₛ) being <1% and 95^{th} percentile of residual measurements being <3ms).

The FHR 10 is displayed together with uterine activity 12. Here, although the FHR 10 does rise in conjunction with an increase in uterine activity 12 (i.e. contractions), the FHR variation is less than usually expected at this stage. While most experienced obstetricians would be aware that the FHR 10 was abnormal, such a reading would not necessarily trigger other alarms (such as an ST event). Therefore there are occasions when such an abnormal reading would be missed.

Figure 2 shows another example for an abnormal FHR that can sometimes be overlooked upon a visual observation only. This STAN^{®} recording was taken during the final stages delivery and again shows the FHR 20 and uterine activity 22. During this stage of labour it is usual for the FHR 20 to be very erratic and therefore many obstetricians would interpret this reading as normal. Even if other alarms, such a ST events, were raised, confusion over the interpretation of the FHR 20 can lead to delays.

In this case, we have a progressive increase in T/QRS ratio identified by the ST log. The FHR 20 was over-reacting (Alarm reaction) to developing hypoxia. Due to uncertainty over the analysis of the FHR 20, the fetus was born 44 minutes later with signs of oxygen deficiency. Note the marked increase in beat-to-beat variation.

Extreme situations of reduced or increased variability in the heart rate cause problems for interpretation as the above examples show. Using the method described above, the FHR can be separated into a primary component and a residual component.

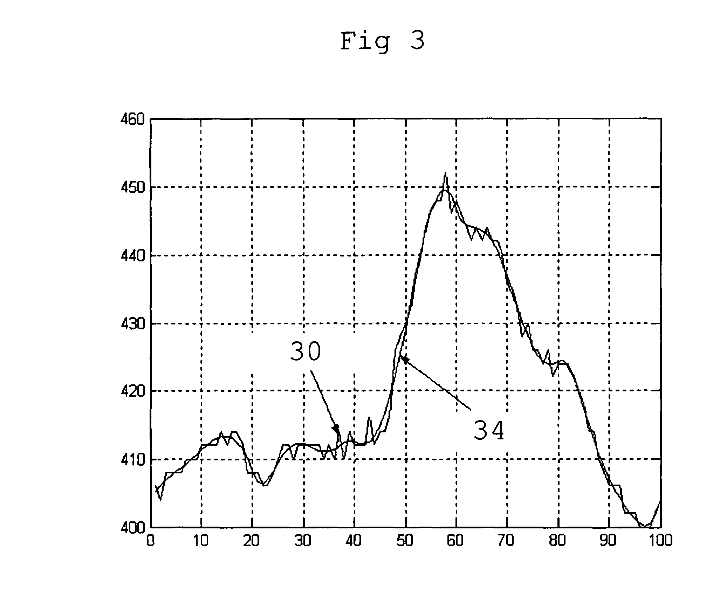

An approximation of the primary FHR component can be calculated by means of polynomial curves fitting. Figure 3 shows a plot of duration (in ms) of consecutive heart beats (RR intervals) over time (in seconds). The polynomial curve fit 34 is shown over the actual RR data 30. Beat-to-beat residual variation corresponds to the difference between the RR data 30 and the curve fit 34 and comprises the FHR residual component. Beat-to-beat variation would correspond to the difference between the RR data and the curve fit.

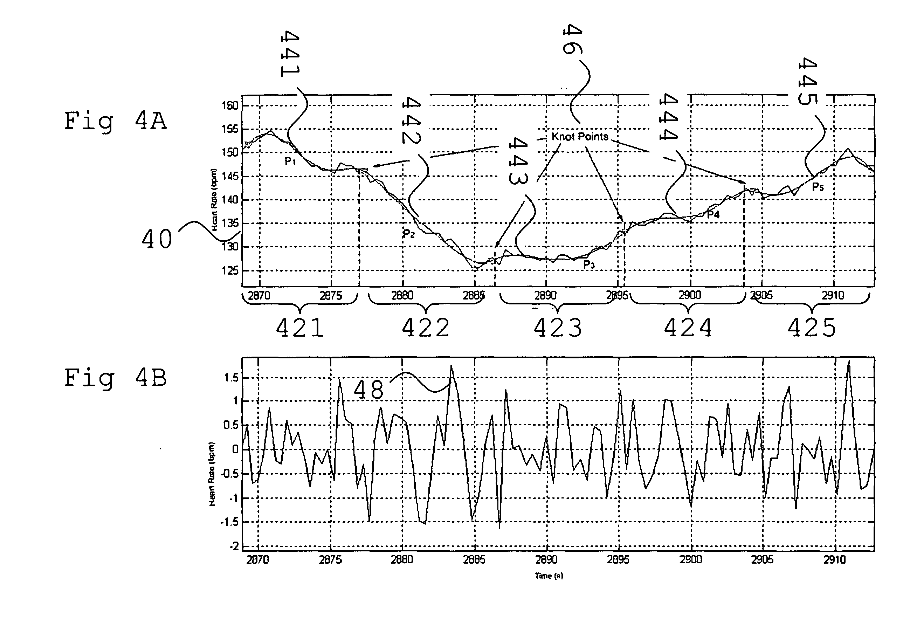

In order to prevent "ringing" in the primary FHR component, the FHR data is divided into small adjacent regions and individual approximations are performed in each region. Figures 4A and 4B show a piecewise polymomial approximation to the HR data. Figure 4A shows a plot of FHR (beats per minute) over time (s). The FHR 40 has been split into 5 adjacent regions 421, 422, 423, 424, 425 of 20 consecutive heart rate samples. A polynomial approximation is carried out in each region 421, 422, 423, 424, 425 to provide individual primary FHR components 441, 442, 443, 444, 445. As the approximations are independent they do not induce "ringing" in the primary FHR component. However, in order to give a smooth continuous curve, it is important to ensure that the primary FHR components 441, 442, 443, 444, 445 align at the boundaries of each region 421, 422, 423, 424, 425. To achieve this, each neighbouring pair of regions shares one heart rate sample. These samples are known as knot points 46. At each knot point 46 the neighbouring polynomial curves are constrained to meet two conditions. Firstly that they have an equal value and secondly that their first derivatives (gradients) are equal. Further constraints can be added so that higher order derivatives must also be equal at the knot points 46, but in this application there is no demonstrated benefit.

Once the primary FHR component has been identified, it is subtracted from the FHR to leave the FHR residual component. Figure 4B shows the FHR residual component 48 which is derived from Figure 4A. Once the FHR residual component 48 has been extracted from the RR or FHR data this can be analysed in a number of ways to monitor the health of the fetus.

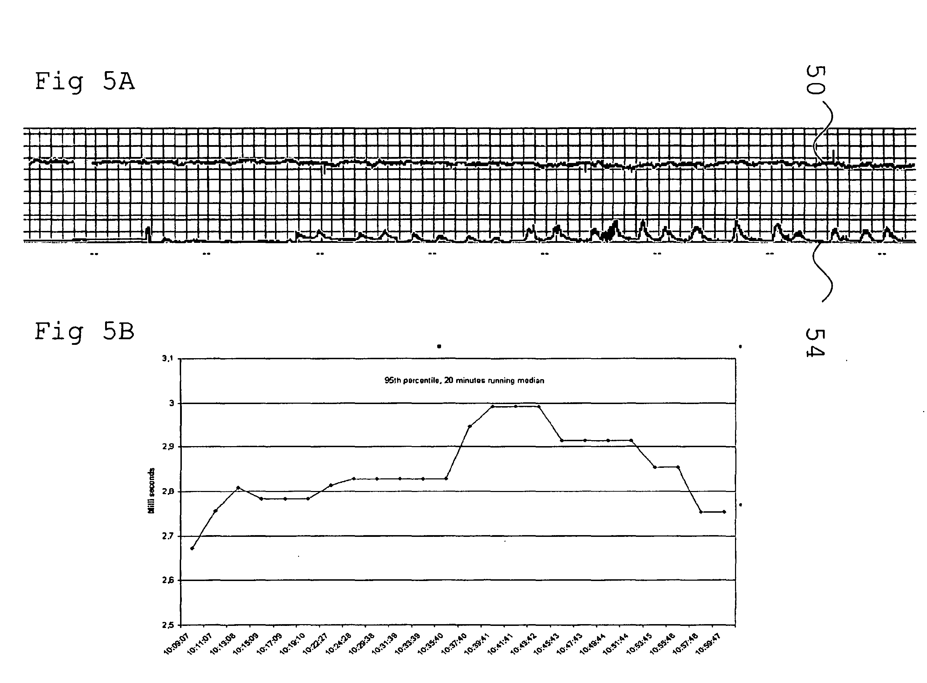

Figure 5A shows the FHR 50 together with the uterine activity 54 in a case of preterminal fetal heart rate pattern recorded during 80 minutes. Figure 5B displays the 95^{th} percentile residual measurements with a 20 minute running median. The 95th percentile shows the level that 95% of the residual component has been below during a certain time period. This reflects changes in the FHR variability and FHR reactivity. From a study of STAN recordings it has been found that a consistent reading of below 3ms, such as that shown in Figure 5B, indicates a lack of reactivity.

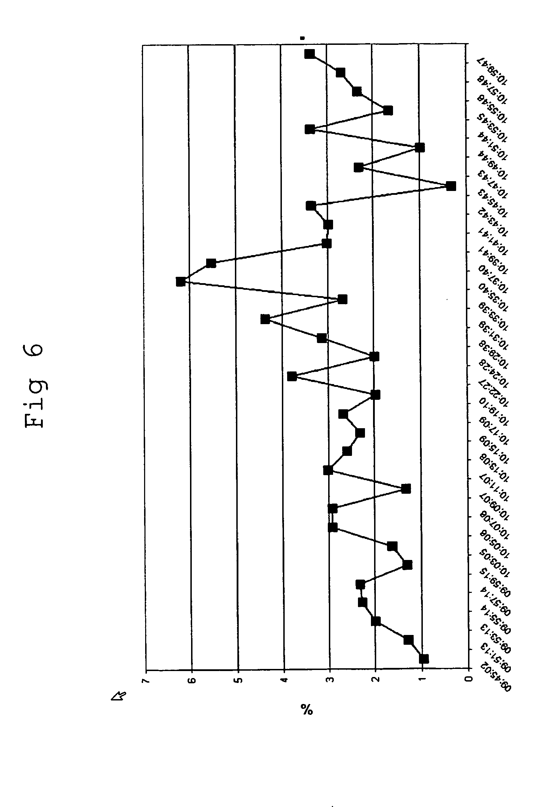

Another area of interest is the 3-4ms frequency distribution. The 3-4ms band has been identified as the highest band which most consistently contains FHR residuals regardless of the fetal heart rate and can thus be used regardless of whether the fetus is in a sleep or active state. It has been found that loss of reactivity can be defined as a situation where the maximum 3-4ms frequency distribution is of <7%. An illustration of this is given in Figure 6 applying the fetal heart rate sequence of Figure 5A.

Some examples, which highlight the benefits of the present invention, are now given.

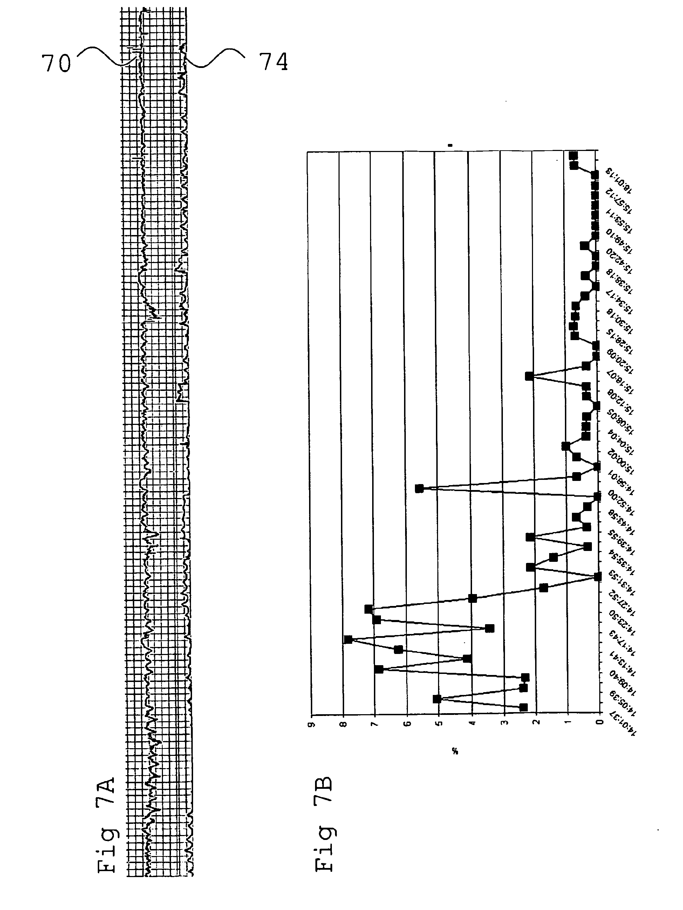

Figure 7A shows the FHR 70 together with the uterine activity 74. In this situation a gradual loss of FHR variation is occurring, which from this data alone can often be missed or at least cause a delay before the loss of reactivity is detected. In this example, the 3-4ms distribution frequency is obtained as described above and this is shown in Figure 7B. It will be seen that in this plot the loss of reactivity is much more apparent and dramatic and therefore easier to detect. With such data available to the obstetrician, diagnosis and action regarding the health of the fetus can be taken more rapidly.

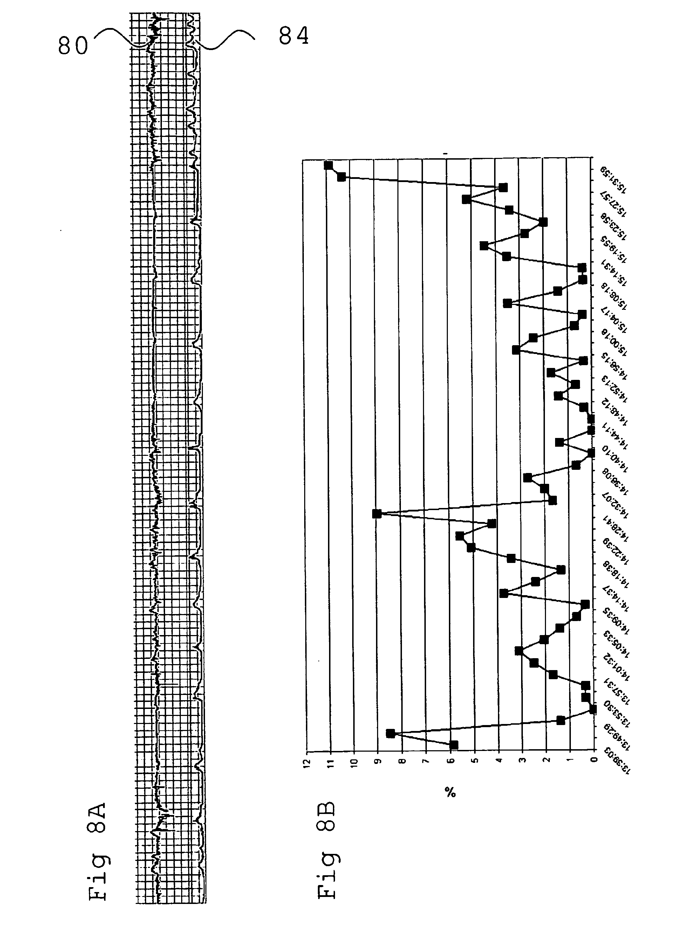

Figure 8A shows another plot of FHR 80, together with uterine activity 84. Here again the FHR variability appears relatively low and from a visual observation may appear to indicate a loss of reactivity. However, from a study of the 3-4ms frequency distribution of the FHR residual component, given in FIG 8B, it is apparent that the residuals are regularly greater than 7%. Therefore, the fetus is healthy and is showing signs of reactivity. Without being able to study this plot, it is possible the obstetrician would make a wrong diagnosis which would lead to unnecessary intervention with the delivery.

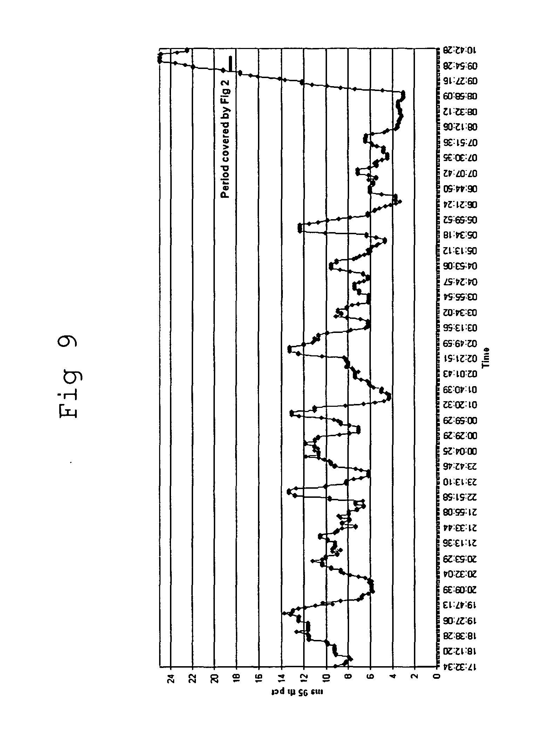

Figure 9 shows the 95th percentile plot of the FHR residual component of FHR 20 recorded in FIG 2. Whereas the FHR readings 20 were indecisive, it is clear from the 95th percentile plot that during the period covered by Figure 2 there is an extreme rise in FHR variability, indicating fetal distress.

The method of the present invention has been applied to stored FHR recordings obtained from thousands of deliveries. Careful study of the FHR residual components in these cases has lead to recommendations for its use according to Table I.

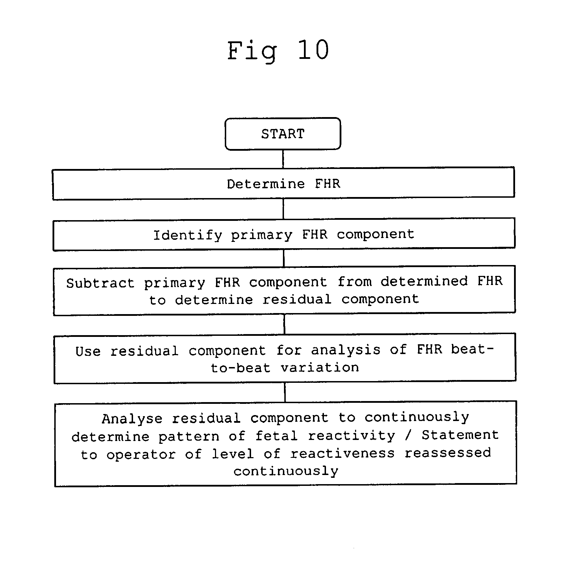

Figure 10 provides a flow chart indicating the different steps in the residual analysis process, according to one emnbodiment of the present invention.

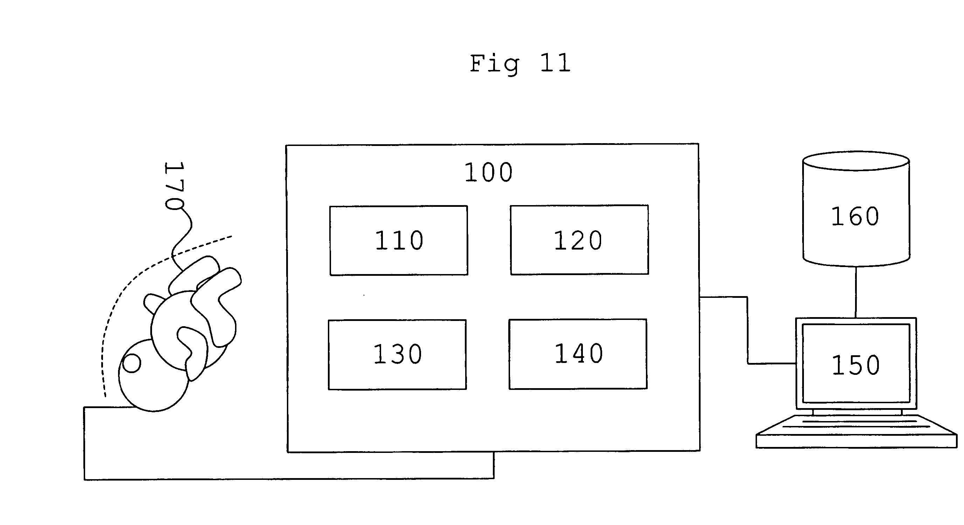

Figure 11 is a schematic illustration of the apparatus 100 according to one embodiment of the present invention, comprising means for determining a fetal heart rate (110), for a fetus (170), means for identifying a primary fetal heart rate component which is required to shift a volume of blood from the heart to the cardiovascular system (120), means for subtracting the primary component from the determined fetal heart rate to determine a residual component (130), means for using said residual component to estimate the fetal heart rate beat-to-beat variation (140), computer means (150), and data storage means (160).

As discussed above, the present invention provides a new and improved way of analysing and monitoring the FHR. This method can be applied during pregnancy and is particularly useful during labour and delivery, when the FHR is non-stationary. The identification of the primary and residual FHR components is a new concept. Careful study of FHR recordings has revealed the most effective methods of applying statistical analysis to the residual FHR component to provide indications of hypoxia.

Specific embodiments of the invention have now been described. However, it should be pointed out that the present invention is not limited to the realizations described above. Several alternatives are possible, as would be apparent for someone skilled in the art. For example, the implementation of the method as discussed above could be accomplished in different ways, such as in especially dedicated hardware or in software, or by a combination ot the two. Further, the means for executing various method steps could be arranged as separate units or entities, but alternatively several method steps could be executed by one single unit. Accordingly, a single unit may perform the functions of several means recited in the claims. Such and other obvious variations must be considered to be part of the present invention, as it is defined in the appended claims.

## Claims

1. An apparatus for fetal monitoring comprising:
a) means for determining a fetal heart rate development over time,
b) means for identifying a primary fetal heart rate component which is required to shift a volume of blood from the heart to the cardiovascular system,
c) means for subtracting the primary component from the determined fetal heart rate to determine a residual component; and
d) means for using said residual component for analysis of the fetal heart rate beat-to-beat variation,
wherein the primary fetal heart rate component is identified through a polynomial curve fit approximation of the fetal heart rate data, and by:
(i) dividing the fetal heart rate data into periods of time of a predetermined size; and
(ii) performing individual polynomial approximations of the fetal heart rate data for each period of time.

2. An apparatus as claimed in claim 1, wherein said means for identifying the primary fetal heart rate component is adapted to perform the following steps:
(i) linear interpolation of recorded fetal heart rate data;
(ii) resampling at a resampling frequency, thereby forming a resampled series of fetal heart rate data, and;
(iii)polynomial curve fit approximation of said resampled series.

3. An apparatus as claimed in claim 1 or 2, wherein the polynomial curve fit approximation utilises polynomials of at least the 5th order.

4. An apparatus as claimed in claim 3, wherein said polynomials are of the 5th order.

5. An apparatus as claimed in claim 3, wherein said polynomials are of the 12th order.

6. An apparatus as claimed in any one of the preceding claims, wherein the polynomial approximation is obtained through a least squares approximation.

7. An apparatus as claimed in any one of the preceding claims, wherein each polynomial approximation is constrained such that neighbouring polynomial functions align and have equal first derivatives at the period border where they join.

8. An apparatus as claimed in any one of the preceding claims, wherein the predetermined size is greater than or equal to a time corresponding to 20 consecutive heart rate samples.

9. An apparatus as claimed in claim 7, wherein the predetermined size is a time corresponding to 20 consecutive heart rate samples.

10. An apparatus as claimed in any preceding claim, wherein the means for using said residual component for analysis of the fetal heart rate beat-to-beat variation is adapted to apply statistical tests for analysing the residual component in order to determine the response of the fetus.

11. An apparatus as claimed in claim 10, wherein the statistical test comprises monitoring of a 95th percentile of the fetal heart rate residual component.

12. An apparatus as claimed in claim 11, wherein the statistical test further comprises calculating a median and a variance of said 95th percentile over a predetermined period of time.

13. An apparatus as claimed in claim 12, wherein said predetermined period of time is longer than 10 minutes.

14. An apparatus as claimed in any one of claims 11 to 13, wherein if the median of the 95th percentile is consistently below 3ms the fetal heart rate is classed as abnormal and non-reactive.

15. An apparatus as claimed in any one of claims 10 to 13, wherein said means for using said residual-component for analysis of the fetal heart rate beat-to-beat variation is adapted to indicate a significant reduction of fetal reactivity given a recording of the median of the 95th percentile below 2.3 ms and the variance of the 95th percentile below 0.1 over an extended period of time.

16. An apparatus as claimed in any one of claims 10 to 13, wherein said means for using said residual component for analysis of the fetal heart rate beat-to-beat variation is adapted to indicate a significant reduction of fetal reactivity given a recording of a decreasing trend of the median of the 95th percentile over an extended period of time.

17. An apparatus as claimed in any one of claims 10 to 13, wherein said means for using said residual component for analysis of the fetal heart rate beat-to-beat variation is adapted to exclude an abnormally low fetal heart rate variation if the median of the 95th percentile is consistently above 3ms.

18. An apparatus as claimed in claim 10, wherein the statistical test comprises monitoring of a short term, e.g. 3-4ms, frequency distribution of the fetal heart rate residual component.

19. An apparatus as claimed in claim 18, wherein if a 3-4ms frequency distribution is less than 7% the fetal heart rate is classed as non-reactive.

20. A method for fetal monitoring comprising the steps of:
a) determining a fetal heart development rate over time,
b) identifying a primary fetal heart rate component which is required to shift a volume of blood from the heart to the cardiovascular system,
c) subtracting the primary component from the determined fetal heart rate to determine a residual component; and
d) using said residual component for analysis of the fetal heart rate beat-to-beat variation,
wherein the primary fetal heart rate component is identified through a polynomial curve fit approximation of the fetal heart rate data, and by:
(i) dividing the fetal heart rate data into periods of time of a predetermined size; and
(ii) performing individual polynomial approximations of the fetal heart rate data for each period of time.

21. A computer program for executing the steps of claim 20 when the programme is executed in a programmable apparatus according to claim 1.

## Patentansprüche

1. Gerät für das Überwachen eines Fötus, mit:
a) Mitteln, um eine zeitliche Entwicklung der fötalen Herzfrequenz zu bestimmen,
b) Mitteln, um eine primäre Komponente der fötalen Herzfrequenz zu identifizieren, die erforderlich ist, um ein Blutvolumen vom Herzen zum kardiovaskulären System zu bewegen,
c) Mitteln, um die primäre Komponente von der ermittelten fötalen Herzfrequenz zu subtrahieren und **dadurch** eine Restkomponente zu bestimmen; und
d) Mitteln, um die Restkomponente für eine Analyse der Intervall-Variation der Schläge der fötalen Herzfrequenz zu nutzen,
**dadurch gekennzeichnet, dass** die primäre Komponente der fötalen Herzfrequenz durch eine polynomische Kurvenapproximation der fötalen Herzfrequenzdaten identifiziert wird, und indem:
(i) die fötalen Herzfrequenzdaten in Zeitabschnitte einer vorbestimmten Länge unterteilt werden; und
(ii) individuelle polynomische Approximationen der fötalen Herzfrequenzdaten für jeden Zeitabschnitt ausgeführt werden.

2. Gerät nach Anspruch 1, **dadurch gekennzeichnet, dass** die Mittel, die primäre Komponente der fötalen Herzfrequenz zu identifizieren, dafür geeignet sind, die folgenden Schritte auszuführen:
(i) lineare Interpolation von aufgezeichneten fötalen Herzfrequenzdaten;
(ii) Ziehung von Stichproben bei einer Stichproben-Frequenz, um **dadurch** eine neue Stichprobenreihe von fötalen Herzfrequenzdaten zu bilden; und
(iii) polynomische Kurvenapproximation der neuen Stichprobenreihe.

3. Gerät nach Anspruch 1 oder 2, **dadurch gekennzeichnet, dass** Polynome mindestens fünfter Ordnung für die polynomische Kurvenapproximation verwendet werden.

4. Gerät nach Anspruch 3, **dadurch gekennzeichnet, dass** die Polynome fünfter Ordnung sind.

5. Gerät nach Anspruch 3, **dadurch gekennzeichnet, dass** die Polynome zwölfter Ordnung sind.

6. Gerät nach einem der vorangehenden Ansprüche, **dadurch gekennzeichnet, dass** die polynomische Approximation durch eine Approximation kleinster Quadrate gewonnen wird.

7. Gerät nach einem der vorangehenden Ansprüche, **dadurch gekennzeichnet, dass** benachbarte polynomische Funktionen jeder polynomischen Approximation bewusst aneinander gereiht werden und an den Zeitabschnittsgrenzen, wo sie zusammentreffen, gleiche erste Ableitungen besitzen.

8. Gerät nach einem der vorangehenden Ansprüche, **dadurch gekennzeichnet, dass** die vorbestimmte Länge mehr als die Zeit beträgt, die 20 aufeinander folgenden Herzfrequenz-Stichproben entspricht, oder gleich dieser Zeit ist.

9. Gerät nach Anspruch 7, **dadurch gekennzeichnet, dass** die vorbestimmte Länge eine Zeit ist, die 20 aufeinander folgenden Herzfrequenz-Stichproben entspricht.

10. Gerät nach einem der vorangehenden Ansprüche, **dadurch gekennzeichnet, dass** die Mittel zur Nutzung der Restkomponente für eine Analyse der Intervall-Variation der Schläge der fötalen Herzfrequenz dafür geeignet sind, statistische Prüfungen anzuwenden, um die Restkomponente zu analysieren und die Reaktion des Fötus zu ermitteln.

11. Gerät nach Anspruch 10, **dadurch gekennzeichnet, dass** die statistische Prüfung die Überwachung eines Prozentranges 95 der Restkomponente der fötalen Herzfrequenz umfasst.

12. Gerät nach Anspruch 11, **dadurch gekennzeichnet, dass** die statistische Prüfung weiter die Berechnung eines Medians und einer Varianz des Prozentranges 95 über eine vorbestimmte Zeitperiode hinweg umfasst.

13. Gerät nach Anspruch 12, **dadurch gekennzeichnet, dass** die vorbestimmte Zeitperiode länger als 10 Minuten ist.

14. Gerät nach einem der Ansprüche 11 bis 13, **dadurch gekennzeichnet, dass** die fötale Herzfrequenz als abnormal und nichtreaktiv beurteilt wird, wenn der Median des Prozentranges 95 stetig unterhalb von 3 ms liegt.

15. Gerät nach einem der Ansprüche 10 bis 13, **dadurch gekennzeichnet, dass** die Mittel zur Nutzung der Restkomponente für eine Analyse der Intervall-Variation der Schläge der fötalen Herzfrequenz dafür geeignet sind, eine signifikante Verringerung der fötalen Reaktivität anzuzeigen, wenn über eine verlängerte Zeitperiode hinweg der Median des Prozentranges 95 mit weniger als 2,3 ms und die Varianz des Prozentranges 95 mit weniger als 0,1 verzeichnet werden.

16. Gerät nach einem der Ansprüche 10 bis 13, **dadurch gekennzeichnet, dass** die Mittel zur Nutzung der Restkomponente für eine Analyse der Intervall-Variation der Schläge der fötalen Herzfrequenz dafür geeignet sind, eine signifikante Verringerung der fötalen Reaktivität anzuzeigen, wenn über eine verlängerte Zeitperiode hinweg ein Abwärtstrend des Medians des Prozentranges 95 verzeichnet wird.

17. Gerät nach einem der Ansprüche 10 bis 13, **dadurch gekennzeichnet, dass** die Mittel zur Nutzung der Restkomponente für eine Analyse der Intervall-Variation der Schläge der fötalen Herzfrequenz dafür geeignet sind, eine abnormal niedrige Intervall-Variation der Schläge der fötalen Herzfrequenz auszuschliessen, wenn der Median des Prozentranges 95 stetig oberhalb von 3 ms liegt.

18. Gerät nach Anspruch 10, **dadurch gekennzeichnet, dass** die statistische Prüfung die Überwachung einer kurzzeitigen Frequenzverteilung von z.B. 3 bis 4 ms in der Restkomponente der fötalen Herzfrequenz umfasst.

19. Gerät nach Anspruch 18, **dadurch gekennzeichnet, dass** die fötale Herzfrequenz als nicht reaktiv beurteilt wird, wenn eine 3- bis 4-ms-Frequenzverteilung weniger als 7 % beträgt.

20. Verfahren zum Überwachen eines Fötus mit den Schritten:
a) Bestimmung einer zeitlichen Entwicklung der fötalen Herzfrequenz,
b) Identifizierung einer primären Komponente der fötalen Herzfrequenz, die erforderlich ist, um ein Blutvolumen vom Herzen zum kardiovaskulären System zu bewegen,
c) Subtraktion der primären Komponente von der ermittelten fötalen Herzfrequenz, um eine Restkomponente zu bestimmen; und
d) Nutzung der Restkomponente für eine Analyse der Intervall-Variation der Schläge der fötalen Herzfrequenz,
**dadurch gekennzeichnet, dass** die primäre Komponente der fötalen Herzfrequenz durch eine polynomische Kurvenapproximation der fötalen Herzfrequenzdaten identifiziert wird, und indem:
(i) die fötalen Herzfrequenzdaten in Zeitabschnitte einer vorbestimmten Länge unterteilt werden; und
(ii) individuelle polynomische Approximationen der fötalen Herzfrequenzdaten für jeden Zeitabschnitt ausgeführt werden.

21. Computerprogramm für die Ausführung der Schritte des Anspruchs 20, wenn das Programm in einem programmierbaren Gerät nach Anspruch 1 abläuft.

## Revendications

1. Appareil de contrôle foetal comprenant:
a) un moyen pour déterminer un développement de la fréquence cardiaque foetale au cours du temps,
b) un moyen pour identifier une composante principale de la fréquence cardiaque foetale qui est nécessaire pour déplacer un volume de sang du coeur au système cardiovasculaire,
c) un moyen pour retrancher la composante principale de la fréquence cardiaque foetale déterminée pour déterminer une composante résiduelle; et
d) un moyen pour utiliser ladite composante résiduelle pour l'analyse de la variation battement par battement de la fréquence cardiaque foetale,
où la composante principale de la fréquence cardiaque foetale est identifiée par le biais d'une approximation d'ajustement de courbes polynomiales des données de la fréquence cardiaque foetale, et en:
i) divisant les données de la fréquence cardiaque foetale en périodes temporelles d'une taille prédéterminée; et
ii) effectuant des approximations polynomiales individuelles des données de fréquence cardiaque foetale pour chaque période de temps.

2. Appareil comme revendiqué dans la revendication 1, dans lequel ledit moyen pour identifier la composante principale de la fréquence cardiaque foetale est adapté pour effectuer les étapes suivantes:
i) une interpolation linéaire de données de fréquence cardiaque foetale enregistrées;
ii) un ré-échantillonnage à une fréquence de ré-échantillonnage, formant ainsi une série ré-échantillonnée de données de fréquence cardiaque foetale, et
iii) une approximation d'ajustement de courbes polynomiales de ladite série ré-échantillonnée.

3. Appareil comme revendiqué dans la revendication 1 ou 2, dans lequel l'approximation d'ajustement de courbes polynomiales utilise des polynômes d'au moins le 5ième ordre.

4. Appareil comme revendiqué dans la revendication 3, dans lequel lesdits polynômes sont du 5ième ordre.

5. Appareil comme revendiqué dans la revendication 3, dans lequel lesdits polynômes sont du 12ième ordre.

6. Appareil comme revendiqué dans l'une quelconque des revendications précédentes, dans lequel l'approximation polynomiale est obtenue par le biais d'une approximation des moindres carrés.

7. Appareil comme revendiqué dans l'une quelconque des revendications précédentes, dans lequel chaque approximation polynomiale est contrainte de sorte que des fonctions polynomiales voisines s'alignent et ont des dérivées premières égales au niveau de la frontière périodique où elles se rejoignent.

8. Appareil comme revendiqué dans l'une quelconque des revendications précédentes, dans lequel la taille prédéterminée est supérieure ou égale à un temps correspondant à 20 échantillons de fréquence cardiaque consécutifs.

9. Appareil comme revendiqué dans la revendication 7, dans lequel la taille prédéterminée est un temps correspondant à 20 échantillons de fréquence cardiaque consécutifs.

10. Appareil comme revendiqué dans l'une quelconque des revendications précédentes, dans lequel le moyen pour utiliser ladite composante résiduelle pour l'analyse de la variation battement par battement de la fréquence cardiaque foetale est adapté pour appliquer des essais statistiques pour analyser la composante résiduelle dans le but de déterminer la réponse du foetus.

11. Appareil comme revendiqué dans la revendication 10, dans lequel l'essai statistique comprend le fait de contrôler un 95ième centile de la composante résiduelle de la fréquence cardiaque foetale.

12. Appareil comme revendiqué dans la revendication 11, dans lequel l'essai statistique comprend en plus le fait de calculer une médiane et une variance dudit 95ième centile sur une période de temps prédéterminée.

13. Appareil comme revendiqué dans la revendication 12, dans lequel ladite période de temps prédéterminée est plus longue que 10 minutes.

14. Appareil comme revendiqué dans l'une quelconque des revendications 11 à 13, dans lequel si la médiane du 95ième centile est régulièrement sous les 3 ms, la fréquence cardiaque foetale est déclarée comme anormale et non réactive.

15. Appareil comme revendiqué dans l'une quelconque des revendications 10 à 13, dans lequel ledit moyen pour utiliser ladite composante résiduelle pour analyser la variation battement par battement de la fréquence cardiaque foetale est adapté pour indiquer une réduction significative d'une réactivité foetale dans le cas d'un enregistrement de la médiane du 95ième centile sous les 2,3 ms et la variance du 95ième centile en dessous de 0,1 sur une période de temps étendue.

16. Appareil comme revendiqué dans l'une quelconque des revendications 10 à 13, dans lequel ledit moyen pour utiliser ladite composante résiduelle pour analyser la variation battement par battement de la fréquence cardiaque foetale est adapté pour indiquer une réduction significative d'une réactivité foetale dans le cas d'un enregistrement d'une tendance décroissante de la médiane du 95ième centile sur une période de temps étendue.

17. Appareil comme revendiqué dans l'une quelconque des revendications 10 à 13, dans lequel ledit moyen pour utiliser ladite composante résiduelle pour analyser la variation battement par battement de la fréquence cardiaque foetale est adapté pour exclure une variation de la fréquence cardiaque foetale anormalement basse si la médiane du 95ième centile est régulièrement supérieure à 3 ms.

18. Appareil comme revendiqué dans la revendication 10, dans lequel l'essai statistique comprend le fait de contrôler une distribution de fréquence à court terme, par exemple 3-4 ms, de la composante résiduelle de la fréquence cardiaque foetale.

19. Appareil comme revendiqué dans la revendication 18, dans lequel si une distribution de fréquence de 3-4 ms est inférieure à 7% la fréquence cardiaque foetale est déclarée comme non réactive.

20. Procédé de contrôle foetal comprenant les étapes de:
a) déterminer un développement de la fréquence cardiaque foetale au cours du temps,
b) identifier une composante principale de la fréquence cardiaque foetale qui est nécessaire pour déplacer un volume de sang du coeur au système cardiovasculaire,
c) retrancher la composante principale de la fréquence cardiaque foetale déterminée pour déterminer une composante résiduelle; et
d) utiliser ladite composante résiduelle pour l'analyse de la variation battement par battement de la fréquence cardiaque foetale,
où la composante principale de la fréquence cardiaque foetale est identifiée par le biais d'une approximation d'ajustement de courbes polynomiales des données de fréquence cardiaque foetale, et en:
i) divisant les données de fréquence cardiaque foetale en périodes de temps d'une taille prédéterminée, et
ii) effectuant des approximations polynomiales individuelles des données de fréquence cardiaque foetale pour chaque période de temps.

21. Programme informatique pour exécuter les étapes de la revendication 20 lorsque le programme est exécuté dans un appareil programmable selon la revendication 1.
